# EUROPEAN PATENT APPLICATION

(11) **EP 4 781 950 A1**
(43) Date of publication of application: **29.07.2026**
(21) Application number: 25154425.0
(22) Date of filing: 28.01.2025
(51) Int. Cl.: A61F 2/26

(54) **A PENILE IMPLANT REAR TIP EXTENDER WITH LOCKING TEETH CONFIGURED TO RADIALLY BALANCE ENGAGEMENT FORCES BETWEEN THE REAR TIP EXTENDER AND THE PENILE IMPLANT**

(71) Applicant: Coloplast A/S, 3050 Humlebaek (DK)
(72) Inventor: ALLEN, John J., Mendota Heights, 55118 (US); Cornelius, Linda, Minneapolis, 55411 (US); Sleeva, Charles, Minneapolis, 55401 (US)

(57) **Abstract**

A rear tip extender (104) attachable to a penile implant a first tooth (180) with a first tooth bite edge (202) offset radially inward from a cavity sidewall and a second tooth (182) with a second tooth bite edge (212) offset radially inward from the cavity sidewall (196). A leading end of the first tooth bite edge and a leading end of the second tooth bite edge are each an equal distance away from a distal end of the rear tip extender.

## Description

### Background

An implanted penile prosthetic is effective in relieving male erectile dysfunction.

A penile prosthesis (a device is a prosthesis prior to implant and a prosthetic after implantation) could be a malleable and non-inflatable device or an inflatable device.

The malleable device has a flexible core that is bendable to various orientations, which for example allows the user to bend the penile implant to a desired erect shape or to a relaxed state.

The inflatable device typically includes a pair of cylinders implantable in the penis, a reservoir implantable in the abdomen, and a pump implantable in the scrotum that is employed to move liquid from the reservoir into the cylinders. Pumping liquid from the reservoir to the cylinders inflates the penile implant to allow for penetrative intercourse.

Each of these penile prostheses has a distal portion that is implantable within a dilated corpus cavernosum and a proximal portion that is implantable within a dilated crus penis. There can be variations in the depth and width of the dilated crus penis, even bi-laterally within an individual patient. The surgeon desires to appropriately fit the proximal portion of the penile prosthesis within the crus and might desire to attach length-extenders to the implant to adjust for the depth of the crus penis. These length-adjusting accessories should be easy to attach precisely, yet resistant to detaching from the implant in the case of explant.

Surgeons and users would benefit from devices that improve the fit of an implantable penile prosthesis within the penis.

### Brief Description of the Drawings

The accompanying drawings are included to provide a further understanding of embodiments and are incorporated in and constitute a part of this specification. The drawings illustrate embodiments and together with the description teach principles of this disclosure. Other embodiments and the intended advantages of embodiments will be readily appreciated as they become better understood by reference to the following detailed description. The elements of the drawings are not necessarily to scale relative to each other. Like reference numerals designate corresponding similar parts.
Fig. 1 is a cross-sectional view of a prior art rear tip extender.
Fig. 2 is a perspective view of one embodiment of a penile implant system including a rear tip extender attachable to one of two penile implants.
Fig. 3 is a side view of one of the penile implants of Fig. 2.
Fig. 4A is a longitudinal cross-sectional view of a left half and Fig. 4B is a longitudinal cross-sectional view of a right half of the rear tip extender of Fig. 2 showing, in combination, two teeth.
Fig. 5 is a view of one embodiment of a rear tip extender including multiple teeth and a snap ring, where the rear tip extender has been splayed open to show the entire interior sidewall of the cavity of the rear tip extender.
Fig. 6 is a side view of one embodiment of a penile implant useful with the system of Fig. 2, with the penile implant including a lock groove and a snap groove.
Fig. 7 is a side view of another embodiment of a rear tip extender.
Fig. 8 is a cross-sectional view of the rear tip extender of Fig. 7 provided with a cavity sized to receive an annular lock ring.
Fig. 9 is a perspective view of one embodiment of an annular lock ring configured to be inserted into the cavity of Fig. 8.
Fig. 10 is a bottom side view of the annular lock ring of Fig. 9.
Fig. 11 is a side view of the annular lock ring of Fig. 9.
Fig. 12 is a cross-sectional view of the annular lock ring of Fig. 9.
Fig. 13 is a perspective view of another embodiment of an annular lock ring configured to be inserted into the cavity of Fig. 8.
Fig. 14 is a bottom side view of the annular lock ring of Fig. 13.
Fig. 15 is a side view of the annular lock ring of Fig. 13.
Fig. 16 is a cross-sectional view of the annular lock ring of Fig. 13.
Fig. 17 is a perspective view of another embodiment of an annular lock ring configured to be inserted into the cavity of Fig. 8.
Fig. 18 is a bottom side view of the annular lock ring of Fig. 17.
Fig. 19 is a side view of the annular lock ring of Fig. 17.
Fig. 20 is a cross-sectional view of the annular lock ring of Fig. 17.
Fig. 21 is a cross-sectional view of the rear tip extender of Fig. 8 including the annular lock ring of Fig. 17 engaged with a proximal portion of the penile implant of Fig. 6.
Fig. 22 is a schematic view of the system of Fig. 2 implanted in a patient.

### Summary

A surgeon might decide during a surgical procedure that a penile implant will fit the patient better if the length of the implant is increased. In such a case, the surgeon will have incised the penis of the patient, dilated each corpus cavernosum of the penis, and measured both corpora to determine a suitable the implant length. The penis is thus ready to receive a properly sized implant, which is determined by the surgeon after taking the steps described above. If the surgeon decides to use a rear tip extender, he or she will secure an appropriately sized rear tip extender to the proximal end of each penile prosthesis.

A problem has been identified that when a rear tip extender is attached to a penile prosthesis, for example by twisting the rear tip extender into place, the alignment of the rear tip extender can be off axis (or crooked) relative to the implant, and this can result in a misalignment between the two parts and a potentially weaker retention force between the rear tip extender and the prosthesis. If the surgeon determines that the alignment of the attached rear tip extender could be improved, the surgeon will remove the rear tip extender and try to re-attach the rear tip extender with an improved alignment. Attaching and subsequently detaching a misaligned rear tip extender can be difficult and is undesirably time consuming.

We have discovered this problem can be solved by providing the rear tip extender with an improved engagement feature that consistently engages the prothesis in an aligned manner by balancing the radial engagement forces of the rear tip extender around a proximal base of the prosthesis. One example of this solution is to provide the rear tip extender with multiple engagement teeth where each tooth engages (starts to bite) at an approximately equal longitudinal distance around the proximal base of the prosthesis. The engagement of the teeth at approximately equal longitudinal distances, and at approximately the same time radially around the proximal base of the prosthesis, operates to radially balance the engagement forces between the rear tip extender and the penile implant. The engagement teeth in the rear tip extender engage at different locations around the tapered proximal base of the prothesis, but at about the same distance along the prosthesis, to radially balance the engagement forces between the rear tip extender and the penile implant.

The solution described below includes a rear tip extender provided with teeth, where each tooth has a base (where the base connects the tooth to the rear tip extender or to a lock ring inside of the rear tip extender) and the bases are in the same row (or planar) equidistantly from an end of the rear tip extender. Each tooth extends from the base to a leading bite edge, and the leading bite edges are oriented at preferably the same pitch. The pitch of the tooth can be viewed as an angle of the leading bite edge relative to its base, for example. Specifically, a leading end of a first tooth bite edge and a leading end of a second tooth bite edge (and a leading end of a next tooth bite edge, if provided) are located an equal distance away from a distal end of the rear tip extender such that all teeth (two, three, or four, or more etc.) of the rear tip extender engage with the implant at the same time. The leading ends of the bite edges of the teeth contact the prosthesis and engage at nearly identical locations longitudinally along the prosthesis. Thus, as the rear tip extender is seated on the prosthesis and twisted into engagement, each tooth engages at about the same time and at about the same longitudinal location resulting in balanced radial forces of the teeth biting into the prosthesis to thus provide better alignment and improved retention of the rear tip extender to the prosthesis.

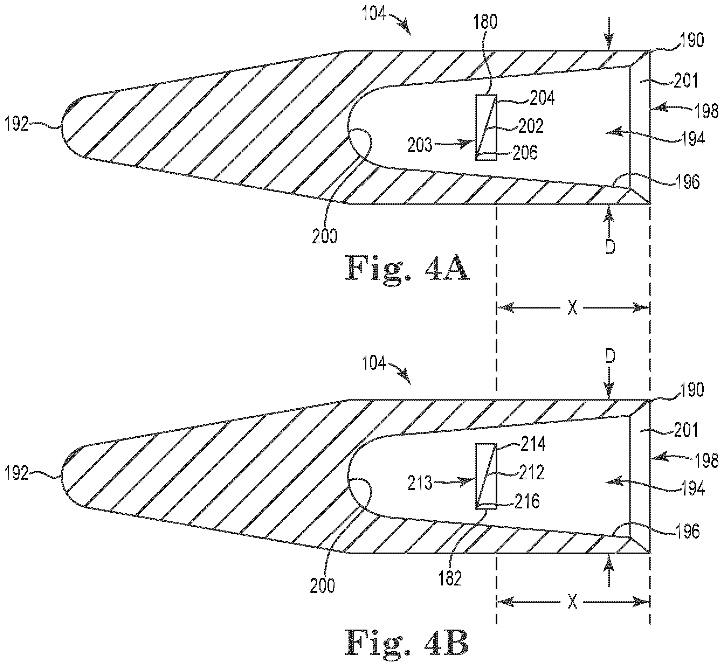

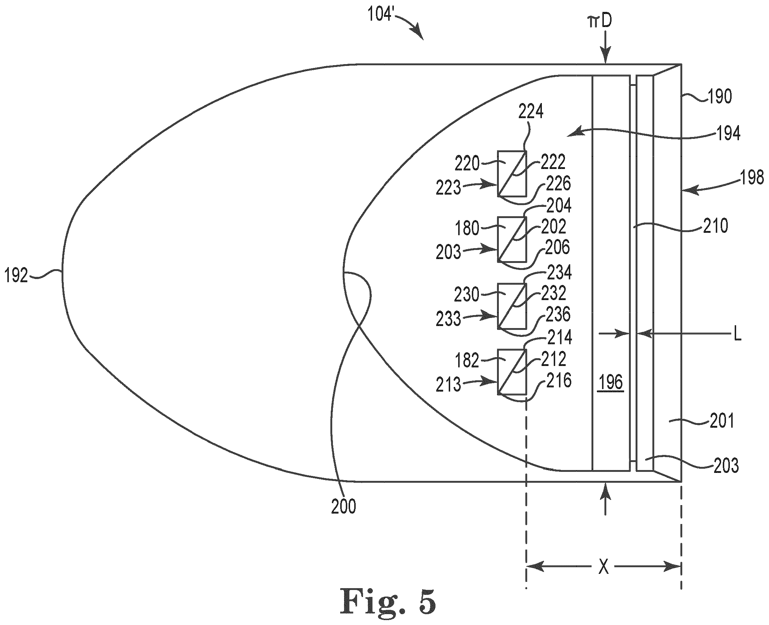

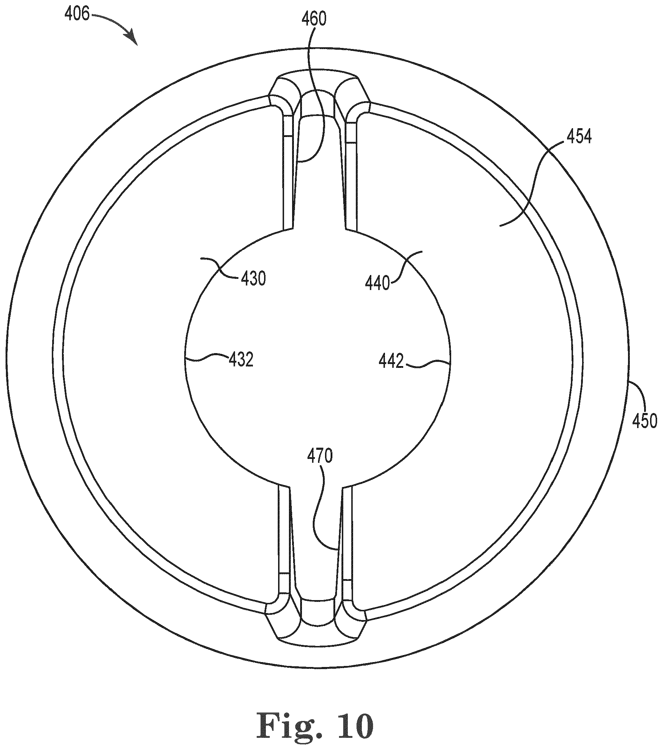

The teeth described are integrated with the rear tip extender in some embodiments (see, for example, Figs. 4A-4B and 5), and in other embodiments, the teeth are provided on a lock ring that is retained inside of the rear tip extender (see, for example, Figs. 10, 14, and 18). The teeth, whether of the integrated style or the lock ring style, operate first to balance radial forces of the teeth as they engage with the prosthesis to thus provide better alignment and improved retention of the rear tip extender to the prosthesis, and second, to provide improved engagement of the rear tip extender to the prosthesis to resist separation of the rear tip extender from the prosthesis if explantation of the device is desirable.

In addition to the pitch of the leading bite edges of the teeth discussed above, each tooth can also be angled to lean toward a distal end of the rear tip extender or toward a proximal end of the rear tip extender. Preferably, the teeth are angled to lean toward the proximal end, or the rear end, of the rear tip extender as described below.

Each tooth is, in a sense, cantilevered from a peripheral wall in a radial inward direction inside of the rear tip extender. Further, the teeth are structured to repose at an angle that projects the bite edge of each tooth in a proximal direction. The teeth angled in the proximal direction advantageously resist removal of the rear tip extender from the penile prosthesis. When the prothesis is pulled in a distal direction (for example during explantation of the implant), the pulling force is effectively applied against each cantilevered tooth in a distal direction. The deflection of the bite edges from a predominantly proximal orientation toward a distal orientation in response to the distal pulling force causes the cantilevered teeth to rotate distally and inwardly, and thus to more aggressively pinch down onto the prosthesis. Consequently, the rear tip extenders described herein offer improved resistance to separation from the prosthesis during an explantation procedure.

Rear tip extenders allow a surgeon to custom-fit the length and width of the proximal end of the prosthesis to the size of the dilated crus opening. The surgeon first determines if the procedure would be improved by attaching a rear tip extender to the penile prosthesis. If the surgeon determines that to be the case, a rear tip extender is selected and fitted to the proximal end portion of the prosthesis. Usually, the rear tip extender is pushed onto the prosthesis, often with a twisting motion that threads the rear tip extender in place for implantation. The rear tip extender will flex a small amount in a radial direction as it engages with the conically shaped proximal portion of the prosthesis.

The rear tip extender described in this patent application includes a spacing or gap distance provided between adjacent teeth of the rear tip extender to accommodate the flexing of the rear tip extender as it bites into and is pulled "upward" on the conical surface of the penile implant. The configuration of the teeth, and specifically the position of the leading bite edge of each tooth, improves the alignment and engagement of the rear tip extender to the prosthesis.

The rear tip extender described in this patent application has locking teeth configured to radially balance engagement forces between the rear tip extender and the penile implant to ensure improved alignment and improved retention.

The applicants describe below suitable such rear tip extenders that may be employed both with a malleable penile prosthesis that is not liquid inflatable, for example the malleable penile prosthesis sold by Coloplast A/S under the trademark GENESIS^{®}, as well as penile prostheses that are inflatable, for example the inflatable penile prosthesis sold by Coloplast A/S under the trademark TITAN^{®}.

The applicants have determined and described below suitable such rear tip extenders having a plurality of spaced apart engagement teeth. One embodiment described below provides the rear tip extender with two spaced apart engagement teeth. Other embodiments described below provide the rear tip extender with more than two spaced apart teeth, one example of which is a rear tip extender with three engagement teeth, although a rear tip extender with four or more teeth is also acceptable.

After reading this disclosure, a person of ordinary skill in the art of penile prostheses will understand that a variety of approaches may be used in implementing aspects of the disclosed rear tip extender. This disclosure is provided to teach several such embodiments of a rear tip extender that are useful, but these examples are provided to further the understanding of the reader and not to limit the scope of this disclosure.

A first exemplary embodiment is a rear tip extender attachable to a penile implant to increase implant length, the rear tip extender comprising:
a cavity formed in the rear tip extender, the cavity comprising a cavity sidewall extending in a proximal direction from an opening formed in a distal end of the rear tip extender to a cavity bottom; and
a plurality of teeth extending radially inward from the cavity sidewall, with the plurality of teeth including a first tooth spaced a distance apart from a second tooth, where:
   the first tooth comprises a first tooth bite edge offset radially inward from the cavity sidewall,
   the second tooth comprises a second tooth bite edge offset radially inward from the cavity sidewall;
wherein each of the first tooth bite edge and the second tooth bite edge comprises a leading end and a trailing end, with the leading end of the first tooth bite edge and the leading end of the second tooth bite edge each positioned an equal distance away from the distal end of the rear tip extender.

An aspect of the above embodiment includes wherein the trailing end of the first tooth bite edge and the trailing end of the second tooth bite edge are each an equal distance away from the distal end of the rear tip extender.

An aspect of the above embodiment includes wherein the leading end of the first tooth bite edge is closer to the distal end of the rear tip extender than is the trailing end of the first tooth bite edge.

An aspect of the above embodiment includes wherein the first tooth comprises a first tooth base secured to the cavity sidewall and the second tooth comprises a second tooth base secured to the cavity sidewall, and the first tooth base and the second tooth base are each an equal distance away from the distal end of the rear tip extender.

An aspect of the above embodiment includes wherein the plurality of teeth further comprises a third tooth spaced a distance apart from the second tooth, where the third tooth comprises a third tooth bite edge offset radially inward from the cavity sidewall, and the leading end of the third tooth bite edge and the leading end of the second tooth bite edge are each an equal distance away from the distal end of the rear tip extender.

An aspect of the above embodiment includes wherein the second tooth comprises a second tooth base secured to the cavity sidewall and the third tooth comprises a third tooth base secured to the cavity sidewall, and the second tooth base and the third tooth base are each an equal distance away from the distal end of the rear tip extender.

An aspect of the above embodiment includes wherein an exterior surface of the rear tip extender extends from the distal end to a proximal end, and the exterior surface comprises a tapered section that converges from a diameter measured at the distal end to a narrower diameter measured at the proximal end.

An aspect of the above embodiment includes wherein the proximal end of the rear tip extender is a hemi-spherical proximal end.

An aspect of the above embodiment further comprises: an annular lock ring integrated in the cavity sidewall, and the plurality of teeth extend radially inward from the annular lock ring and are positioned along the annular lock ring to orient the plurality of teeth to extend radially inward from the cavity sidewall.

An aspect of the above embodiment includes wherein the annular lock ring has an axial length measured between a distal end and a proximal end of the annular lock ring, and the trailing end of the first tooth bite edge and the trailing end of the second tooth bite edge are each located at the proximal end of the annular lock ring.

An aspect of the above embodiment includes wherein the plurality of teeth further comprises a third tooth spaced a distance apart from the second tooth, where the third tooth comprises a third tooth bite edge offset radially inward from the annular lock ring, and the leading end of the third tooth bite edge and the leading end of the second tooth bite edge are each an equal distance away from the distal end of the annular lock ring.

An aspect of the above embodiment includes wherein the annular lock ring further comprises a first slot formed entirely through a wall of the annular lock ring, with the first slot located between the first tooth and the second tooth and extending through the proximal end of the annular lock ring a partial distance toward the distal end of the annular lock ring.

An aspect of the above embodiment includes wherein the annular lock ring further comprises:
a first slot formed entirely through a wall of the annular lock ring between the first tooth and the second tooth;
a second slot formed entirely through a wall of the annular lock ring between the second tooth and the third tooth; and
a third slot formed entirely through a wall of the annular lock ring between the third tooth and the first tooth;
wherein the first slot, the second slot, and the third slot allow each of the first tooth, the second tooth, and the third tooth to move independently one from another.

An aspect of the above embodiment further comprises:
a snap ring projecting radially inward from the cavity sidewall and located between the plurality of teeth and the distal end of the rear tip extender;
wherein the snap ring is disposed around an entire circumference of the cavity sidewall.

An aspect of the above embodiment includes wherein the snap ring is rectangular in cross-sectional profile and projects at a right angle a radial height inward from the cavity sidewall, and the snap ring radial height away from the cavity sidewall is shorter than an axial length of the snap ring longitudinally along the cavity sidewall.

An aspect of the above embodiment includes wherein the first tooth bite edge and the second tooth bite edge are each oriented to have an identical pitch.

An aspect of the above embodiment includes wherein the first tooth bite edge, the second tooth bite edge, and the third tooth bite edge are each oriented to have an identical pitch.

A different exemplary embodiment is directed to a system having an implant and a rear tip extender that is attachable to the implant, specifically: a penile implant system comprising:
a penile implant comprising a proximal portion implantable in a crus penis;
a rear tip extender provided with a cavity and adapted to an axial length of the penile implant; and
a first tooth and a second tooth extending radially inward within the rear tip extender and distributed around a circumference of a sidewall of the cavity;
wherein the first tooth comprises a first tooth bite edge and the second tooth comprises a second tooth bite edge;
wherein, when the proximal portion of the penile implant is inserted into the cavity of the rear tip extender, the first tooth bite edge and the second tooth bite edge engage the penile implant at a same longitudinal location to radially balance engagement forces between the rear tip extender and the penile implant.

An aspect of this added embodiment includes wherein the first tooth bite edge is oriented at a tooth pitch and the second tooth bite edge oriented at the same tooth pitch.

An aspect of this added embodiment includes wherein the first tooth bite edge extends a length between a leading end and a trailing end, and both the leading end and the trailing end are separate from and spaced apart from the second tooth bite edge.

An aspect of this added embodiment further comprises:
an annular lock ring integrated in the sidewall of the cavity;
the first tooth and the second tooth extend radially inward and are distributed around a circumference of the annular lock ring.

An aspect of this added embodiment further comprises:
a third tooth spaced a distance apart from the first tooth and from the second tooth, where the third tooth comprises a third tooth bite edge extending radially inward from the annular lock ring;
wherein leading ends of the first tooth bite edge, the second tooth bite edge, and the third tooth bite edge are each located an equal distance away from a distal end of the annular lock ring.

An aspect of this added embodiment includes wherein trailing ends of the first tooth bite edge, the second tooth bite edge, and the third tooth bite edge are each located at a proximal end of the annular lock ring.

An aspect of this added embodiment includes wherein the annular lock ring further comprises:
a first slot formed entirely through a wall of the annular lock ring between the first tooth and the second tooth;
a second slot formed entirely through a wall of the annular lock ring between the second tooth and the third tooth; and
a third slot formed entirely through a wall of the annular lock ring between the third tooth and the first tooth;
wherein the first slot, the second slot, and the third slot allow each of the first tooth, the second tooth, and the third tooth to move independently one from another.

An aspect of this added embodiment further comprises:
a snap ring disposed around an entire circumference and projecting radially inward from the cavity sidewall, with the snap ring located between the annular lock ring and a distal end of the rear tip extender.

An aspect of this added embodiment includes wherein the proximal portion of the penile implant is tapered and comprises a snap groove sized to receive the snap ring of the rear tip extender.

An aspect of this added embodiment includes wherein the snap ring of the rear tip extender is adapted to provide a haptic response when the snap ring enters the snap groove formed in the proximal portion of the penile implant.

An aspect of this added embodiment includes wherein the penile implant is a malleable penile implant comprising a metal core surrounded by polymer.

An aspect of this added embodiment includes wherein the penile implant is an inflatable penile implant comprising a bladder adapted to contain liquid and a flow path provide to move liquid into the bladder and remove liquid from the bladder.

### Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with reference to the orientation of the Figure(s) being described. Because components of embodiments can be positioned in different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense.

The features of the various exemplary embodiments described in this application may be combined with each other, unless specifically noted otherwise.

The term "proximal" as employed in this application means that part that is oriented closest to a center of the human body. A proximal end is the nearest endmost location of a proximal portion of the thing being described. In one example, the proximal end of the rear tip extender or the proximal end of the penile prosthesis, as the case may be, engage with the crus penis.

The term "distal" as employed in this application means that part that is situated farthest away from the center of the human body. A distal end is the furthest endmost location of a distal portion of a thing being described.

As relates to penile implants, the proximal portion of the penile implant is implanted in the crus penis and the distal portion of the penile implant is implanted in the pendulous (external portion) of the penis.

The length and width of the penile prosthesis that is implanted in the distal portion of the penis is selected by the surgeon after the surgeon measures the length and girth of each dissected corpus cavernosum. Common sizes for the penile prosthesis implanted in each corpus cavernosum are characterized as "standard" or "narrow." The length of the prosthesis implanted in the crus penis may be adjusted by using one or more rear tip extenders that are attached to the proximal portion of the prosthesis.

Surgeons desire rear tip extenders that are easy to attach and accurately align, while being resistant to detaching from the implant if explantation becomes desirable.

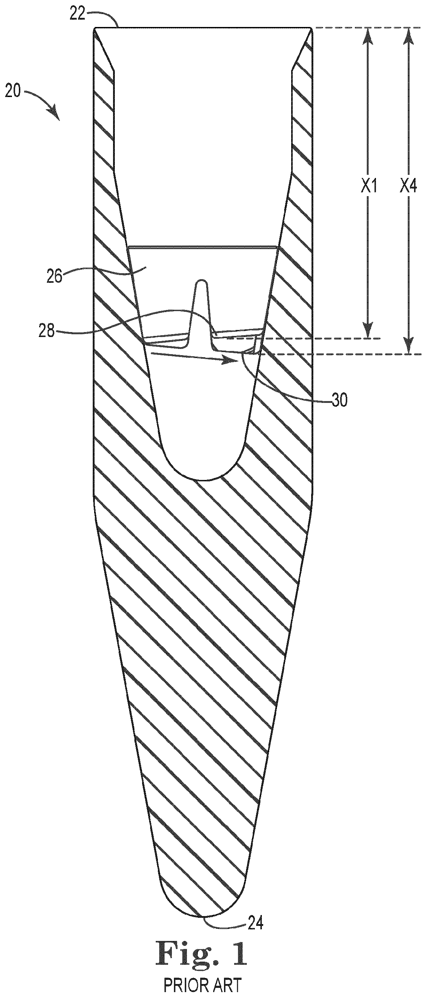

Fig. 1 is a schematic and simplified view of a prior art rear tip extender 20 showing an arrangement of teeth positioned inside of the rear tip extender 20. The prior art rear tip extender 20 has a distal end 22, a proximal end 24, and an insert 26 provided with teeth including a first tooth 28, other teeth, and in this example, a fourth tooth 30. Each of the four teeth of the prior art rear tip extender 20 is located a different distance away from the distal end 22. For example, the first tooth 28 is a distance X1 away from the distal end 22 and the fourth tooth 30 is a distance X4 away from the distal end 22, where the distance X4 is larger than the distance X1. When the prior art rear tip extender 20 is attached to a penile prosthesis, the first tooth 28 contacts the proximal portion of the prothesis first, and then after pushing and twisting the prior art rear tip extender 20 into engagement, the subsequent teeth engage with the prosthesis, and then the fourth tooth 30 eventually contacts the proximal portion of the prothesis. Sometimes the prior art rear tip extender 20 will not align longitudinally with the axis of the prosthesis because the first tooth 28 engages slightly off center and the second, third, and fourth teeth subsequently engage in a similar off-center alignment. While the prior art rear tip extender 20 provides acceptable engagement to the prosthesis, improvements could be made to the alignment of the prior art rear tip extender 20 and the retention force to the prosthesis.

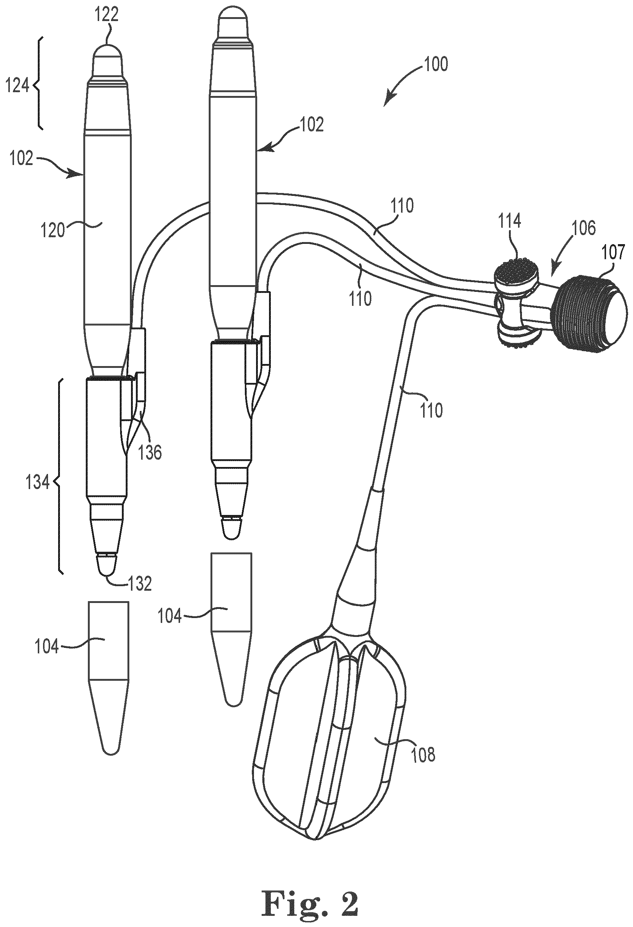

Fig. 2 is a perspective view of one embodiment of a penile implant system 100. The system 100 includes a pair of penile implants 102 each implantable into a corpus cavernosum of the penis and a pair rear tip extenders 104 (RTEs 104) each attachable to one of the penile implants 102. There are two corpus cavernosa in the penis, and thus a pair of penile implants 102 is provided with the system 100. The RTEs 104 advantageously allows a surgeon to selectively increase an axial length of each penile implant 102 that is implanted in the penis, and typically a set of RTEs 104 is provided with the system 100 where the RTEs 104 include a range of lengths (for example, lengths ranging from 2 cm to 5 cm in one-centimeter increments). Each penile implant 102 is referred to as a cylinder, and the cylinder is closed on both ends to allow inflation of the implant 102 with liquid stored in the reservoir 108.

The penile implant system 100 includes a pump 106 attached or attachable between the penile implants 102 and a reservoir 108, for example with suitable tubing 110 connected between the pump 106 and the implants 102 and other like tubing 110 connected between the pump 106 and the reservoir 108.

The pump 106 is provided to move liquid out from the reservoir 108 and into the penile implants 102. One embodiment of the pump 106 includes a pump bulb 107, where squeezing of the pump bulb 107 moves the liquid from the reservoir 108, through the pump 106, and into the implants 102. The displaced liquid is contained under pressure within the implants 102 to provide the implants with a rigidity sufficient for penetrative intercourse. A release pad 114 is provided on the pump 106, where pressing the release pad 114 displaces a valve stem inside the pump 106 to allow the pressurized liquid to return from the penile implants 102 back to the reservoir 108. One suitable pump 106 is available from Coloplast Corp., Minneapolis, Minnesota as part of an erectile dysfunction treatment product sold under the registered trademark TITAN penile prosthesis.

The reservoir 108 is sized to maintain a volume of liquid between about 50-300 ml. In one embodiment, the reservoir 108 is provided as a "cloverleaf" style of reservoir having multiple leaves that may be folded one against the other to compact the reservoir 108 for implantation into the abdomen of the user. One suitable reservoir 108 is sized to retain approximately 130 mL of liquid and is available from Coloplast Corp., Minneapolis, Minnesota.

The RTE 104 is suitable for use with a malleable (or, not inflatable) penile implant or with an inflatable penile implant. The embodiment of the penile implant shown in Fig. 2 is an inflatable penile implant, which is inflatable with liquid from the reservoir 108 to achieve a stiffness suitable for penetrative intercourse. If the surgeon and the patient prefer a malleable penile implant over an inflatable implant, the system 100 of Fig. 1 could be modified to include a malleable implant and the RTE 104.

Each of the penile implants 102 include an inflatable cylinder 120 having a distal end 122 associated with a distal portion 124 of the implant 102 and an end (not visible) that is sealed inside a proximal portion 134 of the penile implant 102. The proximal portion 134 extends to a proximal end 132 of the penile implant 102. A connection tube 136 is connected to the proximal portion 134 of the penile implant 102, where the connection tube 136 is adapted to couple with the tubing 110 and provide a level of strain relief for the implant 102.

The proximal end 132 (if no rear tip extender 104 is attached) is implanted in the crus of the penis and the distal end 122 is implanted within the glans penis. The inflatable cylinders 120 are fabricated from material configured to collapse when the cylinders 120 are deflated to provide the penis with a flaccid state and expand (like a balloon) when the cylinders 120 are inflated with liquid to provide the penis with an erection. As a point of reference, the cylinders 120 shown in Fig. 2 are illustrated in an inflated state having a certain girth. Suitable material for fabricating the cylinders 120 includes silicone, biocompatible polymers such as urethanes, blends of polymers with urethane, copolymers of urethane, or the like.

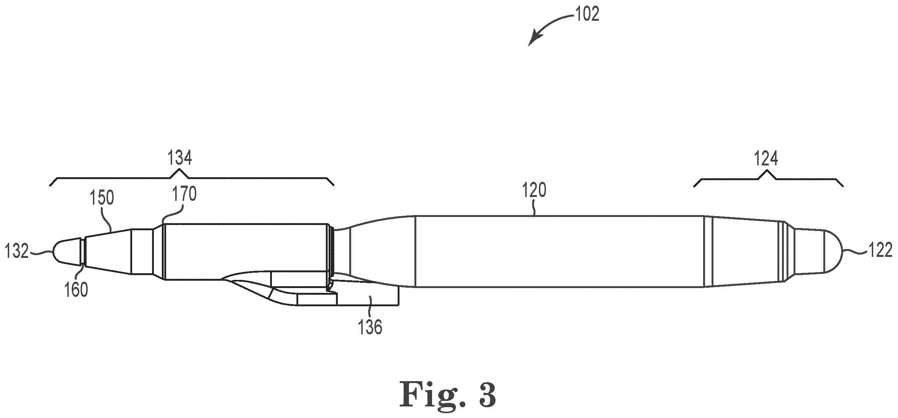

Fig. 3 is a side view of one embodiment of the penile implant 102, where this embodiment illustrates the proximal portion of the implant 102 having an engagement surface adapted to engage with teeth of the RTE 104.

The penile implant 102 extends from the distal end 122 of the distal portion 124 of the implant 102 that is ultimately implanted into the distal part of the penis (the glans penis) to the proximal-most end 132 of the proximal portion 134 of the implant 102 that is ultimately implanted into the proximal part of the penis (the crus penis). The connection tube 136 is connected to the proximal portion 134 of the penile implant 102 and communicates internally with the inflatable cylinder 120.

The proximal portion 134 includes a tapered section 150 having a reduction in diameter converging longitudinally along the proximal portion 134 down to a hemispherical proximal end 132. The illustrated embodiment provides a continuous reduction in diameter for the tapered section 150 toward the proximal-most end 132, although other diameter reduction structures are acceptable. In this embodiment, a lock groove 160 is formed in the tapered section 150 distal of the proximal-most end 132 of the penile implant 102, where the lock groove 160 provides an engagement surface for teeth of the RTE 104. A chamfered section 170 is formed distal the lock groove 160 to allow the RTE 104 to smoothly meet the outside diameter of the proximal portion 134 of the penile implant 102.

The lock groove 160 is a formed into the tapered section 150, where the shape of the lock groove 160 could be one of a variety of shapes, such as a U-shaped groove, a C-shaped groove, or a square-shaped groove with right angles extending upwards from a planar base. The lock groove 160 is adapted to provide a bite surface or ridge for engagement with the teeth of the RTE 104.

Fig. 4A is a cross-sectional view of a left half of the RTE 104 and Fig. 4B is a cross-sectional view of a right half of the RTE 104. The RTE 104 is shown having a cross-sectional cut along a longitudinal centerline to allow a separate view of each half of the RTE 104, and specifically, a view of each tooth 180, 182.

The RTE 104 has multiple teeth that are integrated as a part of a wall of the RTE 104. The multiple teeth are provided to bite into or engage with the proximal portion 134 of the implant 102 (See Fig. 3). Two teeth are described in this embodiment, with a first tooth 180 integrated into the left half and a second tooth 182 integrated into the right half of the RTE 104.

The RTE 104 extends a length between a distal end 190 and a proximal end 192 and includes a cavity 194 formed by a cavity sidewall 196. The cavity 194 extends in a proximal direction from an opening 198 formed in the distal end 190 to a cavity bottom 200. The cavity 194 includes a chamfered edge 201 that allows the RTE 104 to smoothly blend with an outside diameter of the proximal portion 134 of the implant 102 (See Fig. 3).

A plurality of teeth extends radially inward from the cavity sidewall 196, and in this example two teeth 180, 182 extend radially inward. The tooth 180 is formed on the left side of the RTE 104 and the second tooth 182 is formed on the right side of the RTE 104 such that the first tooth 180 is spaced a radial distance apart from the second tooth 182. In one embodiment, the first tooth 180 is spaced about 180 radial degrees from the second tooth 182.

The first tooth 180 has a first tooth bite edge 202 displaced or offset radially inward from a base 203 of the tooth 180 that is secured or integrated with the cavity sidewall 196. The second tooth 182 has a second tooth bite edge 212 offset radially inward from a base 213 of the tooth 182 that is integrated with the cavity sidewall 196. The bite edges 202, 212 are the portions of the teeth 180, 182 that are closest to the center of the RTE 104. The bases 203, 213 of the teeth 180, 182 are in the background of the view (looking down into the page) and the bite edges 202, 212 extend away from the bases 203, 213 and are thus closer to the viewer, or in the foreground of the illustration. The first tooth bite edge 202 has a leading end 204 and a trailing end 206, and the second tooth bite edge 212 has a leading end 214 and a trailing end 216. The leading end 204 of the first tooth bite edge 202 and the leading end 214 of the second tooth bite edge 212 are each positioned an equal distance X away from the distal end 190 of the RTE 104.

In one embodiment, the bite edges 202, 204 of the teeth 180, 182 are each oriented to have an identical pitch such that the trailing end 206 of the first tooth bite edge 202 and the trailing end 216 of the second tooth bite edge 212 are each an equal distance away from the distal end 190 of the RTE 104.

With the orientation of the bite edges 202, 212 described above, the leading end 204 of the first tooth bite edge 202 is closer to the distal end 190 of the RTE 104 than is the trailing end 206 of the first tooth bite edge 202.

In one embodiment, the bases 203, 213 of the teeth 180, 182 are each an equal distance away from the distal end 190 of the RTE 104. Based on the configuration described above, each base 203, 213 is an equal distance away from the distal end 190 of the RTE 104 such that the bases 203, 213 are arranged in a planar configuration inside of the cavity 194. The teeth 180, 182 of the RTE 104 will engage at the same longitudinal location on the penile prothesis since the leading end 204 of the first tooth bite edge 202 and the leading end 214 of the second tooth bite edge 212 are equally close to the distal end 190 of the RTE 104 and are each closer to the distal end 190 than either of the respective trailing ends 206, 216. The engagement teeth 180, 182 thus engage at different angular locations around the tapered proximal end portion of the prothesis (in this case the locations are spaced by 180 degrees) but at the same location/distance (in this case the distance X) along the prosthesis to radially balance the engagement forces between the rear tip extender and the penile implant.

Fig. 5 is a stylized view of another embodiment of an RTE (identified as RTE 104') provided as an example of an RTE having multiple teeth (more than two, in this case). The RTE 104' is similar to the RTE 104 described in Figs. 4A-4B and includes the distal end 190 and the proximal end 192 with the cavity 194 formed by the cavity sidewall 196. The cavity 194 extends in the proximal direction from the opening 198 formed in the distal end 190 that extends to the cavity bottom 200. The chamfered edge 201 allows the RTE 104 to smoothly blend with an outside diameter of the proximal portion 134 of the implant 102 (See Fig. 3). A flat portion 203 extends between the chamfered edge 201 and a profiled snap ring 210 that is provided to increase a retention force of the RTE 104' to an implant.

The view of Fig. 5 shows the RTE 104' cut through one side of an exterior wall and splayed open to expose the entire (interior) sidewall 196 of the cavity 194 (See Fig. 4A). The view of Fig. 5 is looking down on the entire exposed sidewall 196 such that the width of the view is consequently equal to the circumference of the RTE 104' (where the circumference is equal to pi multiplied by the diameter D).

The RTE 104' has a plurality of teeth including the two diametrically opposed teeth 180, 182 described above and other teeth 220, 230.

Tooth 180 has the base 203 connected to the cavity sidewall 196 and a bite edge 202 extending from the leading end 204 to the trailing end 206.

Tooth 182 has the base 213 connected to the cavity sidewall 196 and a bite edge 212 extending from the leading end 214 to the trailing end 216.

Tooth 220 has the base 223 connected to the cavity sidewall 196 and a bite edge 222 extending from the leading end 224 to the trailing end 226.

Tooth 230 has the base 233 connected to the cavity sidewall 196 and a bite edge 232 extending from the leading end 234 to the trailing end 236.

The profiled snap ring 210 is provided to engage with a groove formed on the penile implant 102. The snap ring 210 projects radially inward from the cavity sidewall 196 and is located between the teeth 180, 182, 220, 230 and the distal end 190 of the RTE 104'. In this embodiment, the snap ring 210 is disposed around an entire circumference of the cavity sidewall 196, as shown. In other embodiments it would be acceptable to have the snap ring 210 provided in discrete increments disposed around portions of the circumference of the RTE 104'.

The snap ring 210 is rectangular in cross-sectional profile and projects at a right angle a radial height inward from the cavity sidewall 196. In this embodiment, a radial height of the snap ring 210 projecting away from the cavity sidewall 196 is shorter than an axial length L of the snap ring 210 measured longitudinally along the cavity sidewall 196.

Note in this generalized embodiment of an RTE provided with several teeth that the base 203, 213, 223, 233 of each of the teeth 180, 182, 220, 230, respectively, is aligned with a common plane an equal distance away from the distal end 190 of the RTE 104' and the leading end 204, 214, 224, 234 of each tooth bite edge 202, 212, 222, 232, respectively, is positioned an equal distance X away from the distal end 190 of the RTE 104'.

Suitable dimensions for the RTE are provided as an example. One suitable RTE has a length of 2 cm and a cavity depth of about 0.8 cm which provides an added effective length Le of 1.2 cm when attached to an implant. A variety of RTEs would typically be provided to accommodate adding effective length Le to the penile implants, where surgeons have expressed a desire for the added effective lengths to be in increments of 0.5 cm. The variety of RTEs are provided to allow the surgeon to adjust the effective lengths of the penile prostheses in increments of, for example, 2 cm, 3 cm, 4 cm, 5 cm, etc. As an example of an RTE adding an effective length of 2 cm to a prosthesis, the outside length of the RTE would be about 4 cm with a cavity depth of about 2 cm which provides an added effective length of 2 cm when attached to the penile prosthesis. The proximal end 192 of the RTE 104' is rounded and has a tip radius of 2.0 mm. The distal end 190 of the RTE 104' has an outside diameter D of about 10 mm and is selected to smoothly match a diameter of the implant measured at the engagement location. Thus, the RTE diameter D measured at the distal end is selected to match the diameter of the proximal end portion 134 of the implant 102

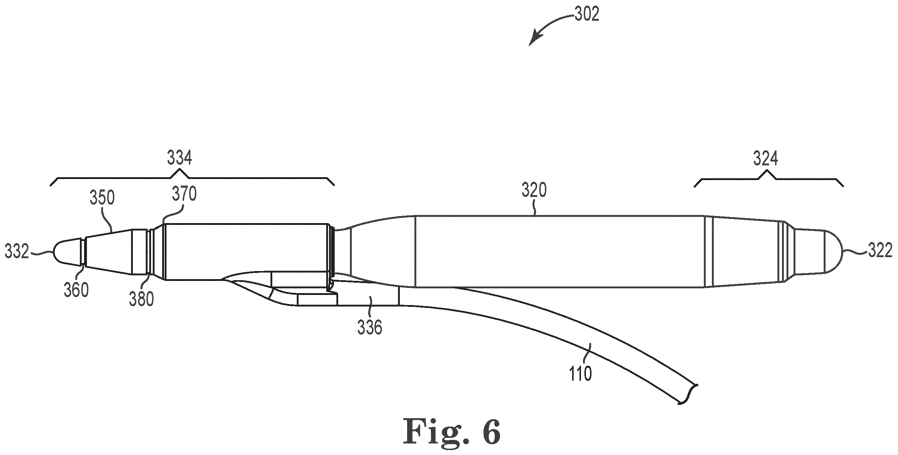

Fig. 6 is a side view of one embodiment of a penile implant 302 adapted for use in the system 100 (See Fig. 2). The penile implant 302 is provided with two engagement surfaces for mating with an RTE, where the engagement surfaces include a lock groove 360 to receive teeth of an RTE and a snap groove 380 to receive a snap ring of an RTE.

The penile implant 302 has an inflatable bladder 320, also called an inflatable cylinder 320 by those of skill in the art. The penile implant 302 extends from a distal end 322 of a distal portion 324 of the implant 302 that is ultimately implanted into the distal part of the penis (the glans penis) to a proximal-most end 332 of a proximal portion 334 of the implant 302 that is ultimately implanted into the proximal part of the penis (the crus penis). The connection tube 336 is connected to the proximal portion 334 of the penile implant 302 and is attachable to the pump 106 (Fig. 2) by the tubing 110, where the connection tube 336 provides a fluid path to the inflatable cylinder 320.

The proximal portion 334 includes a tapered section 350 having a reduction in diameter converging longitudinally along the proximal portion 334 down to the proximal end 332. In this example, the proximal end 332 is a hemispherically rounded end. The illustrated embodiment provides a continuous reduction in diameter for the tapered section 350 toward the proximal-most end 332, although other diameter reduction structures are acceptable.

A lock groove 360 is formed in the tapered section 350 distal of the proximal-most end 332 of the penile implant 302. The lock groove 360 is adapted to engage with teeth of an RTE. The lock groove 360 is a recessed area and is formed in one of a variety of shapes, such as a U-shaped groove, a C-shaped groove, or a square-shaped groove with right angles. The lock groove 360 provides a bite surface for engagement with the teeth of the RTE.

A chamfered section 370 is formed distal the lock groove 360 to allow the RTE to smoothly meet the outside diameter of the proximal portion 334 of the penile implant 302 after the RTE has been coupled to the implant 302.

A snap groove 380 is formed along the surface of the proximal portion 334 of the penile implant 320 and is sized to receive a snap ring provided by the RTE. The snap groove 380 is located between the lock groove 360 and the chamfered section 370. In one orientation, the snap groove 380 is not located on the tapered section 350 but is instead located between the tapered section 350 and the chamfered section 370.

The snap groove 380 is a channel with sharp entrance angles, for example with 90-degree entrance angles leading into the snap groove 380. The snap ring 210, as described above in Fig. 5, has a rectangular cross-sectional profile and projects at a right angle a radial height inward from the cavity sidewall of the RTE.

This configuration of the snap groove 380 and the snap ring of the RTE (described below) configures the combined parts to provide a haptic response when the snap ring enters the snap groove 380 formed in the proximal portion 334 of the penile implant 302. The haptic response can be felt by the gloved fingertips of the surgeon, but the response is also audible for a surgeon having undamaged hearing capable of hearing a sound pitch in a range between 20 - 20,000 hertz, where a typical adult as one example can hear sounds having a frequency of around 15,000 hertz. For such a surgeon capable of hearing sounds of a pitch around 15,000 hertz, the engagement of the snap ring of the RTE into the snap groove 380 provides an audible click or pop indicating that the snap ring has become fully seated in the snap groove 380.

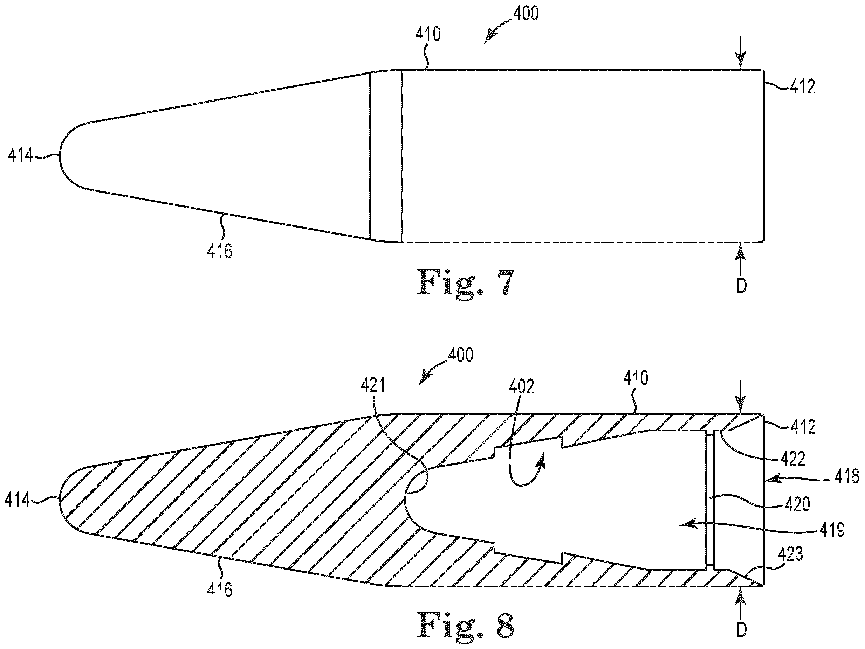

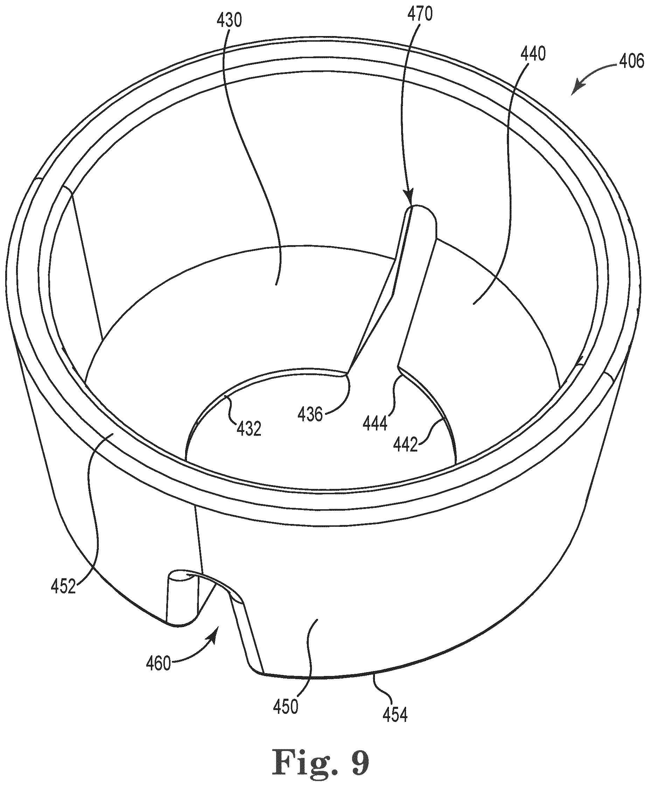

Fig. 7 is a side view of an RTE 400 and Fig. 8 is a cross-sectional view of the RTE 400 provided with an insert mold cavity 402 for a set of several teeth. In one example, the set of teeth is provided as a part of an annular lock ring 406 (Fig. 9) that is insert molded into the mold cavity 402. The RTE 400 is suited for use with the penile implant 302 (Fig. 6).

An exterior surface 410 of the RTE 400 extends from a distal end 412 to a proximal end 414, and the exterior surface 410 has a tapered section 416 that converges from a diameter D measured at the distal end 412 to a narrower diameter measured at the proximal end 414. In one embodiment, the distal end 412 of the RTE 400 includes a tapered flange 423 that is adapted to smoothly mate with the chamfered portion 370 of the implant 302 (Fig. 6). In one embodiment, the proximal end 414 of the RTE 400 is a rounded hemispherical end adapted to fit into a dilated crus penis, although other rounded ends are also acceptable.

The RTE 400 has an opening 418 formed in the distal end 412 that forms a cavity 419 having a cavity sidewall 422 and a cavity bottom 421. The RTE 400 includes an integrated snap ring 420 projecting radially inward from a cavity sidewall 422. The snap ring 420 is located between the teeth and the distal end 412 of the RTE 400. In this embodiment, the snap ring 420 is disposed around an entire circumference of the cavity sidewall 422. The snap ring 420 is rectangular in cross-sectional profile and projects at a right angle a radial height inward from the cavity sidewall 422. In one embodiment, the radial height away from the cavity sidewall 422 is shorter than an axial length of the snap ring 420 longitudinally along the cavity sidewall 422.

In one embodiment, the snap ring 420 is engineered to have a stiffness different from the stiffness of the proximal portion 334 of the implant 302 (Fig. 6). For example, the snap ring 420 is formed of a material, such as silicone, to be stiffer than the material of the proximal portion 334 of the implant 302. In this example, the Shore A durometer of the proximal portion 334 of the implant 302 is selected to be in a range of about 15-40 Shore A and the durometer of the snap ring 420 is selected to be in a range of greater than 40 Shore A to about 70 Shore A. The stiffer snap ring 420 will retain its shape as the snap ring 420 is pushed over the sharp entrance angle leading into the relatively softer snap groove 380. The softer material of the snap groove 380 will flex as the stiffer material of the snap ring 420 enters the snap groove 380, and when the snap ring 420 seats into the groove 380, the wall of the snap groove 380 will snap back or flex back into position, which the inventors have observed results in an audible snapping sound that reliably indicates the snap ring 420 is fully seated in the snap groove 380. Without being bound to this theory, the inventors believe the snap occurs due the snap ring 420 becoming stretched and bent when it reaches the snap groove 380, where the sharp transition at the edge of the snap groove 380 causes the snap ring 420 to abruptly straighten, creating an audible shockwave that is heard as a click or snap. Alternatively, the snap sound that was audible during evaluation may have been due to air being forced out of the snap groove resulting in the audible snap from a "burp" of air. The size of the groove may impact the "quality" of the snap. An example of suitable sizing for the snap ring 420 and the groove 380 that provide an audible sound is for the snap ring 420 to have a height of about 0.030 cm (0.012 inches) and a width of about 0.051 cm (0.020 inches) and for the snap groove 380 to have a depth of about 0.030 cm (0.012 inches) and a width of about 0.061 cm (0.024 inches).

The plurality of teeth provided with the RTE may be integrated with a wall of the RTE (for example the RTE 104 described above) or provided separately by one of the annular lock rings described below. The various engagement teeth embodiments, whether of the integrated style or in the annular lock ring style, could be altered to provide the RTE with two, three, four or more engagement teeth.

The RTE 400 is provided with a plurality of teeth, and in the following embodiment, two teeth are provided by an annular lock ring 406 that is molded into the cavity 402 of the RTE 400.

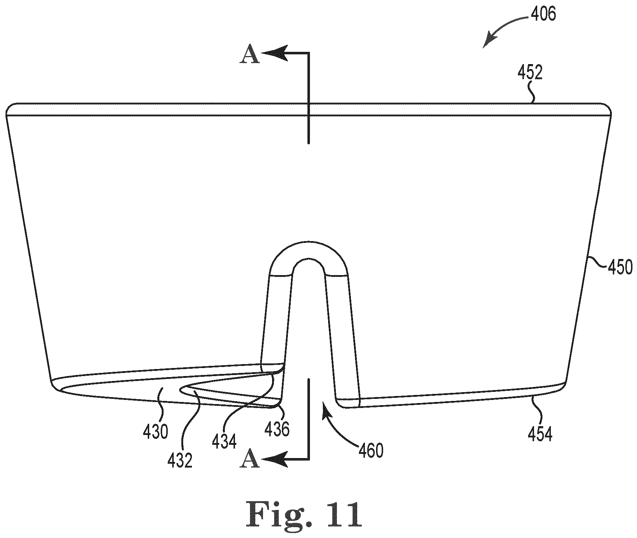

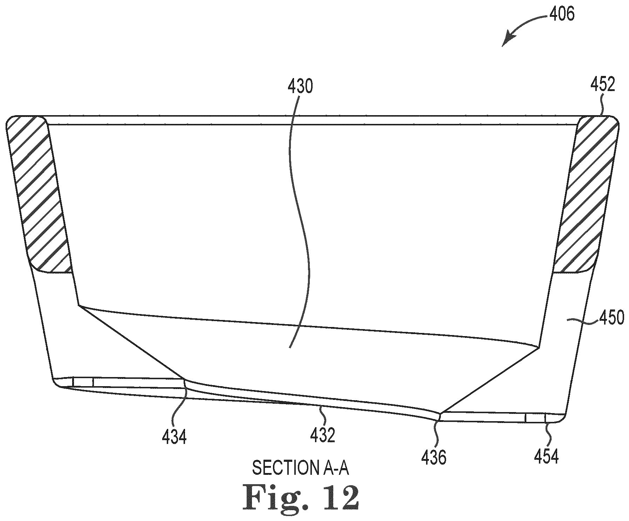

Fig. 9 is a perspective view of one embodiment of the annular lock ring 406 insert moldable into the cavity 402, Fig. 10 is a bottom side view of the annular lock ring 406, Fig. 11 is a side view of the annular lock ring 406, and Fig. 12 is a cross-sectional view of the annular lock ring 406.

The annular lock ring 406 is adapted to be positioned within the cavity 402 formed in the cavity sidewall 422 of the RTE 400 (See Fig. 8). The annular lock ring 406 has a plurality of teeth 430, 440 that extend radially inward from the annular lock ring 406. When the annular lock ring 406 is molded into the RTE 400, the teeth 430, 440 are thus positioned around the annular lock ring 406 and extend radially inward from the cavity sidewall 422.

Each tooth 430, 440 has a bite edge extending between a leading end and a trailing end. The tooth 430 has a tooth bite edge 432 extending between a leading end 434 and a trailing end 436. The tooth 440 has a tooth bite edge 442 extending between a leading end 444 and a trailing end 446. The first tooth bite edge 432 and the second tooth bite edge 442 are each oriented to have an identical pitch.

With reference to Fig. 9 and Figs. 11-12, the first tooth bite edge 432 and the second tooth bite edge 442 are angled at a pitch relative to a distal end 452 of the lock ring 406 and this locates the leading end 434 of the first tooth bite edge 432 and the leading end 444 of the second tooth bite edge 442 an equal distance away from the distal end 452 of the rear tip extender 406, and likewise locates the trailing end 436 of the first tooth bite edge 432 and the trailing end 446 of the second tooth bite edge 442 an equal distance (but different and larger) away from the distal end 452 of the rear tip extender 406.

The annular lock ring 406 has a side wall 450 that defines a length of the annular lock ring 406 measured between a distal end 452 and a proximal end 454. The teeth 430, 434, and in particular the tooth bite edge 432, 442 of each tooth 430, 440, respectively, are located at the proximal end 454 of the annular lock ring 406.

The teeth 430, 440 are separated by a first slot 460 and a second slot 470. Each slot 460, 470 is formed entirely through a thickness of the wall 450 of the annular lock ring 406 and extends through the proximal end 454 of the annular lock ring a partial distance toward the distal end 452 of the annular lock ring 406. It is also acceptable for the slots 460, 470 to be formed only through a portion of the thickness of the wall 450 of the annular lock ring 406.

This embodiment of the annular lock ring 406 is adapted to provide a right-hand thread that will engage with the implant 302 (Fig. 6) when the RTE is turned clockwise. Notably, when the annular lock ring 406 is secured within the RTE 400, the leading end 434 of the first tooth bite edge 432 and the leading end 444 of the second tooth bite edge 442 are each positioned an equal distance away from the distal end 452 of the rear tip extender 400; the trailing end 436 of the first tooth bite edge 432 and the trailing end 446 of the second tooth bite edge 442 are each an equal distance away from the distal end 452 of the rear tip extender 400; and the leading end 434 of the first tooth bite edge 432 is closer to the distal end 452 of the rear tip extender 400 than is the trailing end 436 of the first tooth bite edge 432.

One advantage of providing the teeth 430, 440 on the annular lock ring 406 is that the teeth may be engineered to have a stiffness different from the stiffness of the proximal portion 334 of the implant 302 (Fig. 6). In one example, the teeth 430, 440 of the annular lock ring 406 are formed to be harder than the material of the proximal portion 334 of the implant 302. As an example, the Shore A durometer of the proximal portion 334 of the implant 302 is selected to be in a range of about 15-40 Shore A, the durometer of the teeth provided by the annular lock ring 406 is D on the Shore hardness scale, and the body of the RTE is selected to be in a range of greater than 40 Shore A to about 70 Shore A, for example about 50 Shore A.

With reference to the RTE 400 of Fig. 8, the cavity 402 of the RTE 400 is sized to receive the annular ring 406 in an insert molding process, and the annular ring 406 having two teeth 430, 440 could be used, or a different annular ring may be provided with a wide number of teeth. The next embodiment describes an annular ring provided with three teeth.

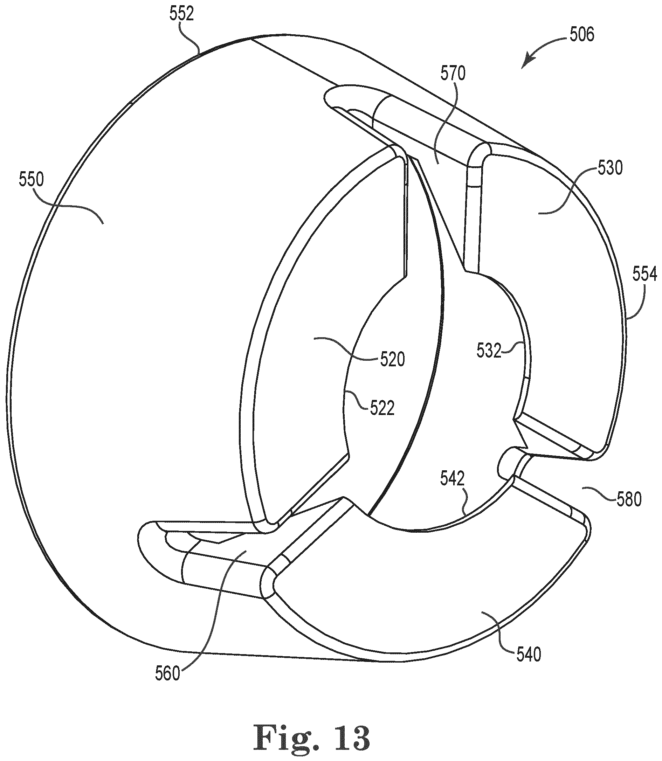

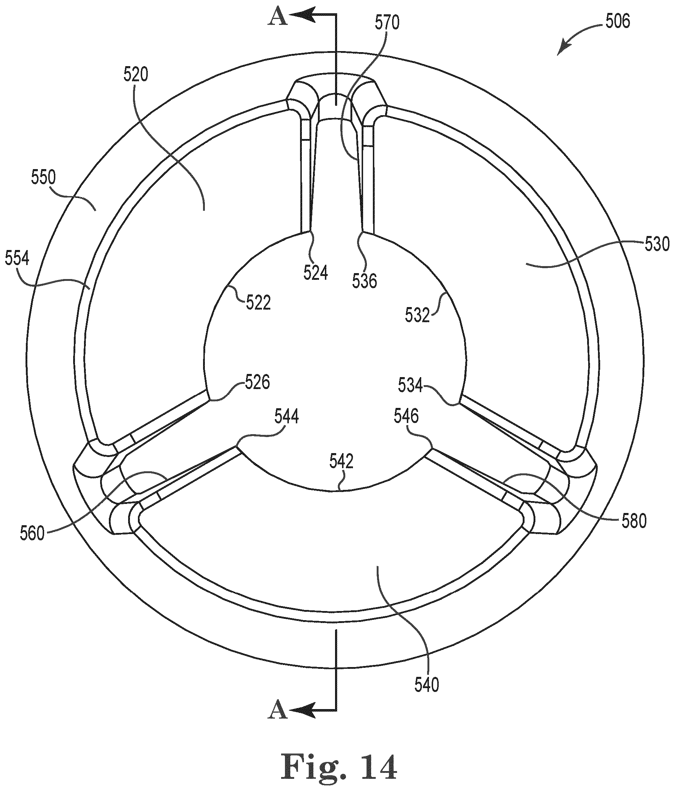

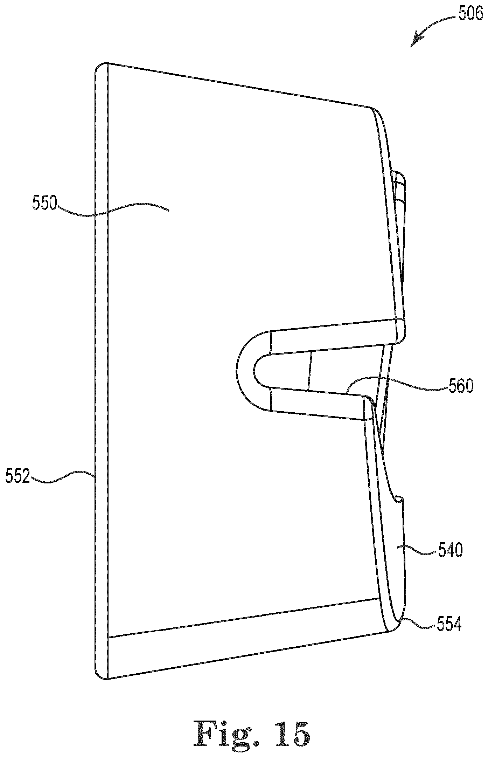

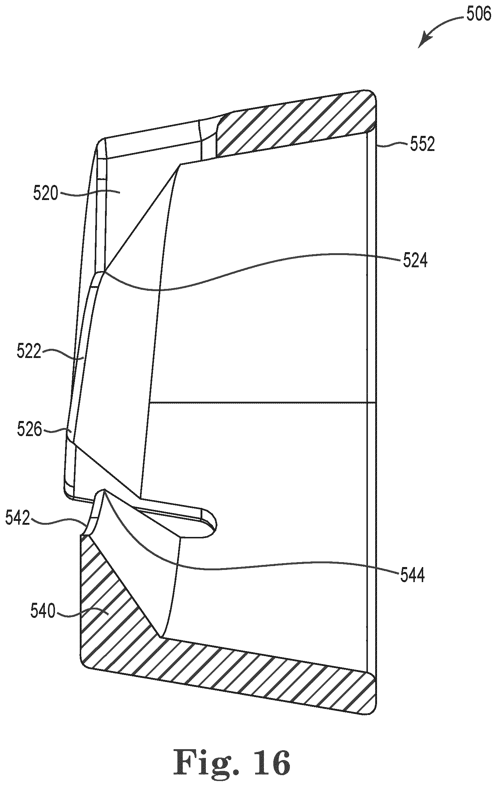

Fig. 13 is a perspective view of one embodiment of an annular lock ring 506 suitable for placement into the cavity 402 (Fig. 8), Fig. 14 is a top side view of the annular lock ring 506, Fig. 15 is a side view of the annular lock ring 506, and Fig. 16 is a cross-sectional view of the annular lock ring 506.

The annular lock ring 506 is configured to be inserted into the cavity 402 formed in the cavity sidewall 422 of the RTE 400 (See Fig. 8) which secures the annular lock ring 506 inside the RTE 400. The annular lock ring 506 has a plurality of teeth 520, 530, 540 that extend radially inward from a sidewall 550 of the annular lock ring 506. When the annular lock ring 506 is molded or inserted into the RTE 400, the teeth 520, 530, 540 are thus positioned around the annular lock ring 506 and extend radially inward from the cavity sidewall 422.

With reference to Fig. 14, each tooth 520, 530, 540 has a bite edge extending between a leading end and a trailing end. The tooth 520 has a tooth bite edge 522 extending between a leading end 524 and a trailing end 526. The tooth 530 has a tooth bite edge 532 extending between a leading end 534 and a trailing end 536. The tooth 540 has a tooth bite edge 542 extending between a leading end 544 and a trailing end 546. The first tooth bite edge 522, the second tooth bite edge 532, and the third tooth bite edge 542 are each oriented to have an identical pitch.

With reference to Fig. 15, the side wall 550 of the annular lock ring 506 defines a length of the annular lock ring 506 measured between a distal end 552 and a proximal end 554. The teeth 520, 530, 540 are located at the proximal end 554 of the annular lock ring 506.

The teeth 520, 530, 540 are separated by a first slot 560, a second slot 570, and a third slot 580 (See Fig. 13). Each slot 560, 570, 580 is formed entirely through a thickness of the wall 550 of the annular lock ring 506 and extends through the proximal end 554 of the annular lock ring 506 and a partial distance toward the distal end 552 of the annular lock ring 506. Fig. 15 shows an embodiment where the sidewalls of each slot 560, 570, 580 are tapered and not parallel. The first slot 560, the second slot 570, and the third slot 580 allow each of the first tooth 520, the second tooth 530, and the third tooth 540 to move independently one from another as the RTE 400 (Fig. 8) and the annular lock ring 506 bite into the proximal end portion 334 of a penile implant 302 (Fig. 6).

The following description relates to teeth having a right-hand pitch that engage with the implant when the RTE is turned clockwise. Those of skill in the art will realize that other teeth having a left-hand pitch could be provided in an RTE that is engaged with an implant when turned counter-clockwise. With this distinction in mind, when the annular lock ring 506 is secured within the RTE 400 (Fig. 8), the leading end 524 of the first tooth bite edge 522, the leading end 534 of the second tooth bite edge 532, and the leading end 544 of the third tooth bite edge 542 are each positioned an equal distance away from the distal end 552 of the rear tip extender 400; the trailing end 526 of the first tooth bite edge 522, the trailing end 536 of the second tooth bite edge 532, and the trailing end 546 of the third tooth bite edge 542 are each an equal distance away from the distal end 552 of the rear tip extender 400; and the leading ends 524, 534, 544 of the teeth bite edges 522, 532, 542 are closer to the distal end 452 of the rear tip extender 400 than the trailing ends 526, 536, 546 of the respective teeth bite edges 522, 532, 542.

The embodiment of Figs. 13-16 has three teeth, where the third tooth 540 is spaced a distance apart from the second tooth 530 and the third tooth bite edge 542 is offset radially inward from the annular lock ring 506. The leading end 544 of the third tooth bite edge 542 and the leading end 534 of the second tooth bite edge 532 are each an equal distance away from the distal end 552 of the annular lock ring 506.

If two teeth are provided on the annular ring, the first tooth bite edge and the second tooth bite edge will each be oriented to have an identical pitch. In the embodiment of Figs. 13-16 provided with three teeth, the first tooth bite edge 522, the second tooth bite edge 532, and the third tooth bite edge 542 are each oriented to have an identical pitch.

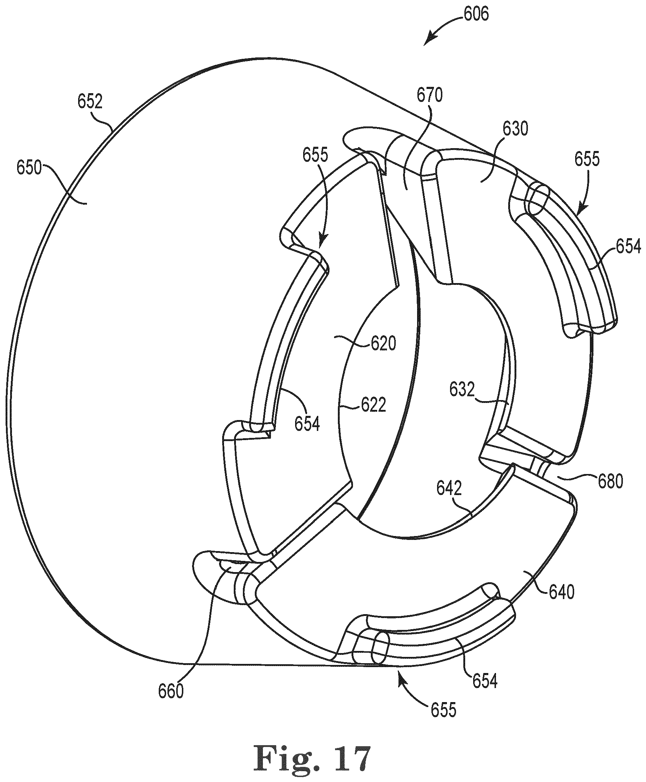

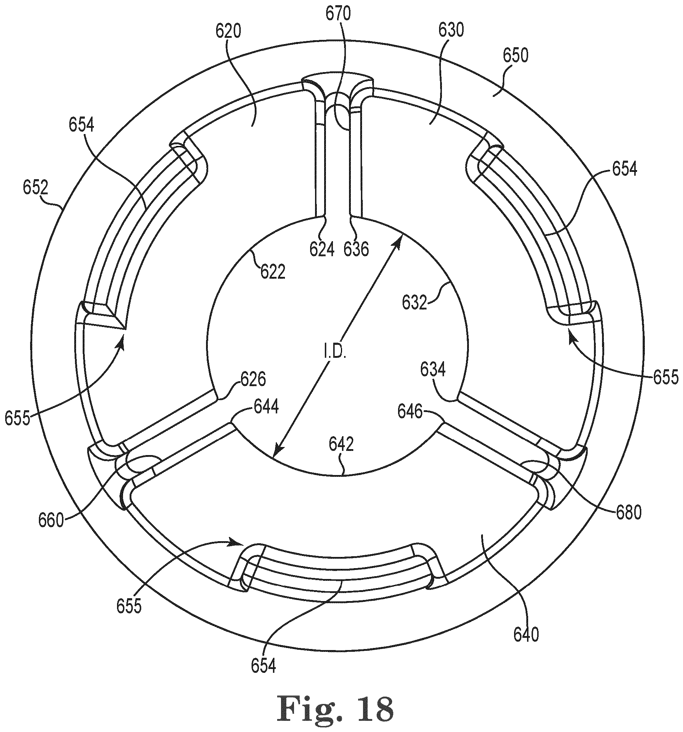

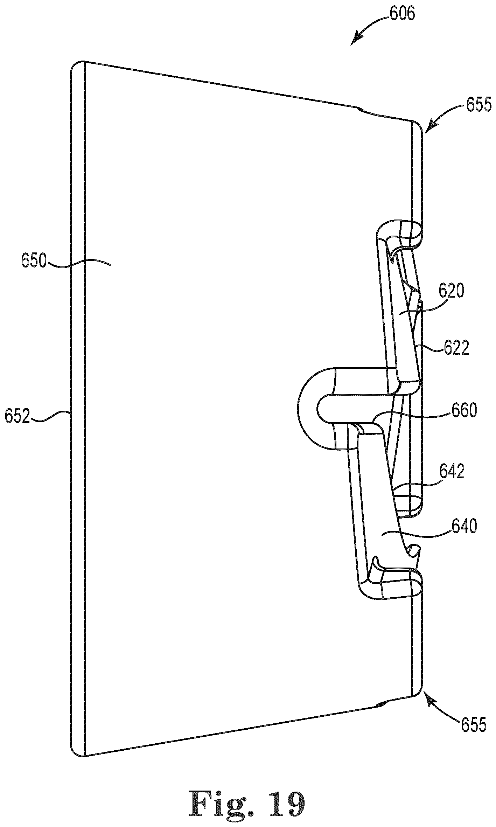

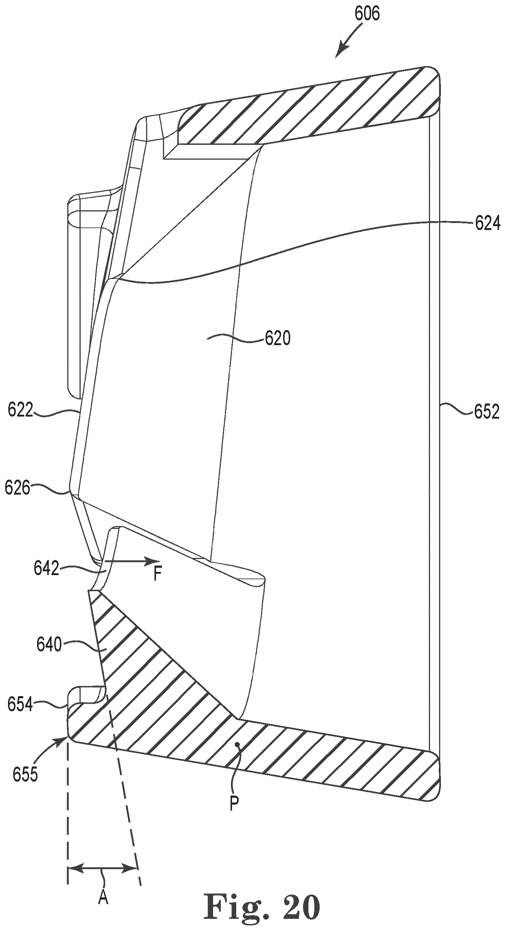

Fig. 17 is a perspective view of another three-tooth annular lock ring 606 insertable into the cavity 402 (Fig. 8), Fig. 18 is a front side view (relative to Fig. 17) of the annular lock ring 606, Fig. 19 is a side view of the annular lock ring 606, and Fig. 20 is a cross-sectional view of the annular lock ring 606. The exemplary three teeth of the annular lock ring 606 have a common pitch and are also disposed at an angle to lean the teeth toward a proximal end of the rear tip extender when the lock ring 606 is retained in the RTE.

The annular lock ring 606 has three teeth 620, 630, 640 that extend radially inward from a sidewall 650 of the annular lock ring 606. When the annular lock ring 606 is molded or inserted into the RTE 400 (Fig. 8), the teeth 620, 630, 640 are angularly distributed around the RTE 400 to extend radially inward from the cavity sidewall 422.

With reference to Fig. 18, each tooth 620, 630, 640 is spaced from an adjacent tooth by a slot 670, 680, 660, respectively, and is provided with a bite edge extending between a leading end and a trailing end. Two differences between the annular lock ring 606 and the annular lock ring 506 are 1) the slots 660, 670, 680 have parallel slot walls and 2) the slots 660, 670, 680 extend a shorted distance compared to slots 560, 570, 580.

The tooth 620 has a tooth bite edge 622 extending between a leading end 624 and a trailing end 626. The tooth 630 has a tooth bite edge 632 extending between a leading end 634 and a trailing end 636. The tooth 640 has a tooth bite edge 642 extending between a leading end 644 and a trailing end 646. The first tooth bite edge 622, the second tooth bite edge 632, and the third tooth bite edge 642 are each oriented to have an identical pitch.

With reference to Fig. 19, the side wall 650 of the annular lock ring 606 defines a length of the annular lock ring 606 measured between a distal end 652 and a proximal end 654, where the proximal end 654 of the lock ring 606 is provided by three standoffs 655. One standoff 655 is formed on an outermost perimeter of each of the three teeth 620, 630, 640. The teeth share a common pitch and the teeth 620, 630, 640 are additionally angled in a proximal direction toward the standoffs 655 and away from the distal end 652 (shown as angle A in Fig. 20). The standoffs 655 ensure that the lock ring 606, when inserted into the cavity 402 (Fig. 8), will be seated in the RTE 400 in a way to encourage the angled teeth 620, 630, 640 to positively engage with the lock groove 360 of the implant 302 (Fig. 6).

The teeth 620, 630, 640 are separated by a first slot 660, a second slot 670, and a third slot 680 (See Figs. 17-18). Each slot 660, 670, 680 is formed entirely through a thickness of the wall 650 of the annular lock ring 606 and extends a short distance toward the distal end 652 of the annular lock ring 606.

Fig. 19 shows the sidewalls of each slot 660, 670, 680 are parallel, in contrast to the tapered sidewalls of the slots 560, 570, 580 of the other three-tooth of Figs. 13-16. The first slot 660, the second slot 670, and the third slot 680 allow each of the first tooth 620, the second tooth 630, and the third tooth 640 to move independently one from another as the RTE 400 (Fig. 8) and the annular lock ring 606 bite into the proximal end portion 334 of a penile implant 302 (Fig. 6). The angled teeth 620, 630, 640 flex and are displaced from a proximal direction toward a distal direction as the lock ring 606 engages with the implant and this movement of the teeth 620, 630, 640 positively bites and engages with the lock groove 360 of the implant 302.

With reference to Figs. 18-19, one advantage of this orientation of the angled teeth 620, 630, 640 is that a force applied to the lock ring 606, for example when the RTE 400 is being pulled off the base of the implant 302, causes a deflection of the bite edges 622, 632, 642 in a distal direction toward the distal end 652 of the lock ring, which results in the bite edges 622, 632, 642 of the teeth 620, 630, 640 compressing more tightly against the lock groove 360 on the implant 302 (Fig. 6). For example, as the RTE 400 is pulled in a proximal direction off from the base of the implant 302, the implant pushes (or drags or pulls) the teeth 620, 630, 640 in a distal direction (or downwards), and this causes the inner diameter (ID of Fig. 18) to become smaller, therefore digging/biting deeper into the base and gripping the base of the implant 302 more firmly. One theory of the mechanism of this improved gripping action of the lock rings described herein is described in more detail in Fig. 20 below.

The following description relates to teeth having a right-hand pitch that engage with the implant when the RTE is turned clockwise. Those of skill in the art will realize that other teeth having a left-hand pitch could be provided in an RTE that is engaged with an implant when turned counterclockwise. With this distinction in mind, when the annular lock ring 506 is secured within the RTE 400 (Fig. 8), the leading end 524 of the first tooth bite edge 522, the leading end 534 of the second tooth bite edge 532, and the leading end 544 of the third tooth bite edge 542 are each positioned an equal distance away from the distal end 552 of the rear tip extender 400; the trailing end 526 of the first tooth bite edge 522, the trailing end 536 of the second tooth bite edge 532, and the trailing end 546 of the third tooth bite edge 542 are each an equal distance away from the distal end 552 of the rear tip extender 400; and the leading ends 524, 534, 544 of the teeth bite edges 522, 532, 542 are closer to the distal end 452 of the rear tip extender 400 than the trailing ends 526, 536, 546 of the respective teeth bite edges 522, 532, 542.

The embodiment of Figs. 13-16 has three teeth, where the third tooth 540 is spaced a distance apart from the second tooth 530 and the third tooth bite edge 542 is offset radially inward from the annular lock ring 506. The leading end 544 of the third tooth bite edge 542 and the leading end 534 of the second tooth bite edge 532 are each an equal distance away from the distal end 552 of the annular lock ring 506.

Fig. 20 is a cross-sectional view of the annular lock ring 606 showing the tooth 640 angled at an angle A upwards relative to a plane incident to the proximal end 654 of the lock ring 606. The angled teeth 620, 630, 640 of the annular lock ring 606 are angled in the proximal direction in a range of about 5 - 15 degrees, for example about 10 degrees.

The lock rings described in this patent application provide improved resistance to the separation of an attached RTE away from a penile implant, which is particularly useful during an explantation procedure where the surgeon desires to remove the entire RTE / implant assembly as a single unit.

One aspect of the improved resistance to separation of the RTE from the implant due to the improved lock rings is described with reference to the tooth 640 of the lock ring 606. The tooth 640 is shown with the bite edge 642 angled at an angle A away from the distal end 652 and in a direction toward the proximal end 654.

The tooth 640 is cantilevered from a wall of the lock ring 606 and extends in a radial inward direction within the rear tip extender. The tooth 640 is angled with the bite edge 642 leaning in a proximal direction toward the proximal end 654 of the lock ring 606. If the surgeon determines that explantation of the implant is desirable, the prothesis would be pulled in a distal direction out of the crus penis. The lock ring 606 in the rear tip extender resists separation of the RTE away from the implant, and consequently, the pulling force applied to the implant would give rise to a force applied to the tooth 640 (identified as force F) in the distal direction. Since the three teeth 620, 630, 640 are distributed around the lock ring 606, the force F applied against the tooth 640 is also applied to the other teeth 620, 630. The force F in the distal direction is resisted by the lock ring 606 because the bite edges 642 (and 622, 632) lean at the angle A toward the proximal direction. Thus, the response to the distal pulling force during explanation is that the cantilevered tooth 640 (and teeth 620, 630) is deflected a small amount in the distal direction, which causes the tooth 640 to rotate inward about the imaginary point P and more aggressively pinch down onto the prosthesis. Consequently, the rear tip extenders described herein offer improved resistance to separation from the prosthesis during an explantation procedure.

The angled teeth 620, 630, 640 of the annular lock ring 606 provide more "bite" into the base of the implant as the RTE is being pulled off or removed, while also balancing the engagement forces between the rear tip extender supplied with the annular lock ring 606 and a penile implant. When an attempt is made to pull the RTE off of the base of an implant, the base draws or pulls down on the top of each of the angled teeth 620, 630, 640 of the annular lock ring 606. The angled teeth 620, 630, 640 are deflected by a removal force applied by the base, and as the teeth 620, 630, 640 are displaced, the inner diameter I.D. (Fig. 18) of the annular lock ring 606 becomes smaller, therefore causing the teeth 620, 630, 640 to dig or bite more deeply into the base and gripping the implant more firmly.

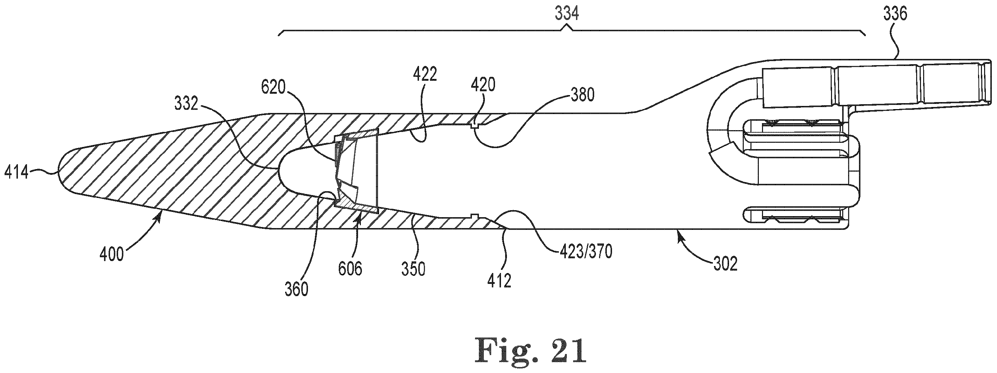

Fig. 21 is a cross-sectional view of the RTE 400 engaged with the proximal portion 334 of the penile implant 302. The RTE 400 is shown with the annular lock ring 606 of Fig. 17 engaged with the proximal portion 334 of the penile implant 302 of Fig. 6. The proximal portion 334 of the penile implant 302 is shown inserted into the cavity sidewall 422 of the RTE 400 with the three teeth 620, 630, 640 (tooth 620 is shown in the cross-section) of the lock ring 606 engaged with the lock groove 360 and the snap ring 420 engaged with the snap groove 380. The tapered flange 423 of the RTE 400 is smoothly mated with the chamfered section 370 of the implant 302 so the outside diameter of the RTE 400 matches with the outside diameter of the implant 302 at that location.

The annular lock ring 606 is provided with three teeth 620, 630, 640, and each of the leading ends 624, 634, 644 of the teeth bite edges 622, 632, 642 are closer to the distal end 412 of the rear tip extender 400 than each of the respective trailing ends of the teeth 620, 630, 640. With this configuration, the leading ends 624, 634, 644 contact the proximal portion 334 of the implant 302 before other portions of the RTE 400. The leading ends 624, 634, 644 of the teeth 520, 530, 540 touch the tapered portion 350 at about the same longitudinal distance along the proximal portion 334 of the penile implant 302, and this balances the radial forces applied to the implant by the RTE 400 as the RT 400 is secured in place. Subsequent twisting or pushing of the RTE 400 onto the penile implant 302 continues to engage the RTE 400 to the implant 302 in a way that maintains the balanced radial forces, which allows the RTE 400 to durably engage with the penile implant 302 and maintain longitudinal alignment with the implant.

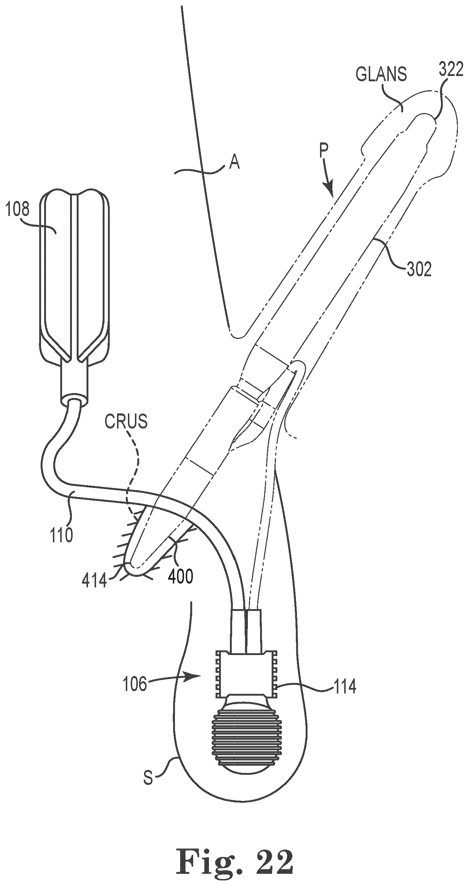

Fig. 22 is a schematic view of the system 100 (Fig. 2) provided with the implant 302 (Fig. 6) and the RTE 400 (Fig. 8) implanted in a patient. The RTE 400 may be fitted with any one of the annular lock rings 406, 506, 606 described above.

The groin area of the patient is shaved and cleaned, for example with a surgical solution, and draped with a sterile drape. A retraction device, such as a surgical retractor sold under the trademark Lone Star and available from Lone Star Medical Products of Stafford, TX, is placed around the penis P. Thereafter, the surgeon forms an incision to access the corpora cavernosa of the penis, where suitable examples of incisions include either an infrapubic incision or a scrotal incision. The infrapubic incision is initiated between the umbilicus and the penis (i.e., above the penis), whereas the transverse scrotal incision is made across an upper portion of the patient's scrotum S. As an example of the transverse scrotal approach, the surgeon forms a 2-3 cm transverse incision through the subcutaneous tissue of the median raphe of the upper scrotum S and dissects down through the Dartos fascia and Buck's fascia to expose the tunicae albuginea of the penis P. Thereafter, each corpus cavernosum is exposed in a corporotomy where a small (approximately 1.5 cm) incision is formed to allow the surgeon to access and subsequently dilate each corpus cavernosum in a distal direction and each crus penis in a proximal direction.

With access to the penis proximal and distal, the surgeon typically will insert an instrument (such as a blunt-ended scissors or other elongated tool) to separate a portion of the spongiosum material to open a path that allows insertion of a device to measure the distal length of each corpus cavernosum. Thereafter, each corpus cavernosum is dilated distally with a suitable reaming tool and each crus is dilated proximally to open the penis and create a space for the implant 302. In one approach, the surgeon begins dilation of the penis by introducing an 8 mm dilator into the spongy tissue of the corpora and the crus with sequential progression to about a 14 mm dilator, each of which are introduced and pushed distally toward the glans penis and then proximally toward the crus of the penis, respectively. After suitable dilation of the space within the penis, the surgeon selects an implant and adjusts the effective length by selecting an appropriately sized RTE.

Embodiments provide an RTE that engages with a rear tip of a penile implant in a way that balances the radial forces of the RTE applied to the implant. For engagement of the RTE 400 to the implant 302, the surgeon will grasp the implant 302 and push the proximal portion 334 of the penile implant 302 into the cavity 422 of the RTE 400 (as one example). The teeth extending radially inward from the cavity sidewall of the RTE engage with the lock groove of the implant, and in the embodiments provided with a snap ring, the snap ring 420 engages with a snap groove formed round the proximal portion of the implant. The snap ring 420 described above will provide an audible sound as the harder snap ring 420 seats into the relatively softer material of the snap groove 380, and this sound indicates tot eh surgeon that the RTE 400 has been fully engaged with the implant 302. The surgeon usually will push the RTE toward the implant while twisting the RTE in a clockwise rotation, and this combined pushing force and twisting motion engages the RTE with the implant. The leading end of the first tooth bite edge and the leading end of the second tooth bite edge are each positioned an equal distance away from the distal end of the rear tip extender, and this configuration ensures that the teeth of the RTE contact at nearly the same time and at about the same longitudinal distance along the implant, so the subsequent engagement forces are balances across each of the several teeth on the RTE. After engagement, the chamfered section at the distal end of the RTE smoothly blends with the outside diameter of the penile implant.

The surgeon inserts and directs the distal end 322 of each of the implants into the distal-most portion of each corpus cavernosum. The proximal end 414 of the RTE 400 is implanted into each of the dilated proximal crus penis. The corporotomy is closed, and the remaining portions of the pump 106 and the reservoir 108 of the system 100 are implanted in the scrotum S and the abdomen A, respectively, of the patient.

The RTE advantageously provides the surgeon with options in adjusting the effective length of the implant. The plurality of teeth within the RTE 104 are positioned to ensure that the engagement forces between the RTE and the implant are radially balanced, which helps to better secure the RTE to the implant and ensures a nearly straight longitudinal alignment of the RTE relative to the implant. Consequently, the RTE remains durably connected to the implant even when explanted.

Embodiments include the following:
A penile implant system comprising:
a penile implant comprising a proximal portion implantable in a crus penis;
a rear tip extender provided with a cavity and adapted to increase an axial length of the penile implant; and
a first tooth and a second tooth extending radially inward within the rear tip extender and distributed around a circumference of a sidewall of the cavity;
wherein the first tooth comprises a first tooth bite edge and the second tooth comprises a second tooth bite edge;
wherein, when the proximal portion of the penile implant is inserted into the cavity of the rear tip extender, the first tooth bite edge and the second tooth bite edge engage the penile implant at a same longitudinal location to radially balance engagement forces between the rear tip extender and the penile implant.

Further wherein the first tooth bite edge is oriented at a tooth pitch and the second tooth bite edge oriented at the same tooth pitch.

Further wherein the first tooth bite edge extends a length between a leading end and a trailing end, and both the leading end and the trailing end are separate from and spaced apart from the second tooth bite edge.

Further including an annular lock ring integrated in the sidewall of the cavity;
the first tooth and the second tooth extend radially inward and are distributed around a circumference of the annular lock ring.

Further including a third tooth spaced a distance apart from the first tooth and from the second tooth, where the third tooth comprises a third tooth bite edge extending radially inward from the annular lock ring;
wherein leading ends of the first tooth bite edge, the second tooth bite edge, and the third tooth bite edge are each located an equal distance away from a distal end of the annular lock ring.

Further wherein trailing ends of the first tooth bite edge, the second tooth bite edge, and the third tooth bite edge are each located at a proximal end of the annular lock ring.

Further wherein the annular lock ring further comprises:
a first slot formed entirely through a wall of the annular lock ring between the first tooth and the second tooth;
a second slot formed entirely through a wall of the annular lock ring between the second tooth and the third tooth; and
a third slot formed entirely through a wall of the annular lock ring between the third tooth and the first tooth;
wherein the first slot, the second slot, and the third slot allow each of the first tooth, the second tooth, and the third tooth to move independently one from another.

Further including a snap ring disposed around an entire circumference and projecting radially inward from the cavity sidewall, with the snap ring located between the annular lock ring and a distal end of the rear tip extender.

Further wherein the proximal portion of the penile implant is tapered and comprises a radial groove sized to receive the snap ring of the rear tip extender.

Further wherein the snap ring of the rear tip extender is adapted to provide a haptic response when the snap ring enters the radial groove formed in the proximal portion of the penile implant.

Further wherein the penile implant is a malleable penile implant comprising a metal core surrounded by polymer.

Further wherein the penile implant is an inflatable penile implant comprising a bladder adapted to contain liquid and a flow path provide to move liquid into the bladder and remove liquid from the bladder.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein.

## Claims

1. A rear tip extender (104, 104', 400) attachable to a penile implant (102, 302) to increase implant length, the rear tip extender comprising:
a cavity (194, 419) formed in the rear tip extender by a cavity sidewall (196, 422) extending in a proximal direction from an opening (198, 418) formed in a distal end (190, 412) of the rear tip extender to a cavity bottom (200, 421); and
a plurality of teeth (180, 182, 220, 230, 430, 440, 520, 530, 540, 620, 630, 640) extending radially inward from the cavity sidewall, with the plurality of teeth including a first tooth (180, 430, 520, 620) spaced a distance apart from a second tooth (182, 440, 530/540, 630/640), where:
the first tooth comprises a first tooth bite edge (202, 432, 522, 622) offset radially inward from the cavity sidewall,
the second tooth comprises a second tooth bite edge (212, 442, 532, 632) offset radially inward from the cavity sidewall;
wherein each of the first tooth bite edge and the second tooth bite edge comprises a leading end (204, 214, 434, 444, 524, 534, 544, 624, 634, 644) and a trailing end (206, 216, 436, 446, 526, 536, 546, 626, 636, 646), with the leading end of the first tooth bite edge and the leading end of the second tooth bite edge each positioned an equal distance (X) away from the distal end of the rear tip extender.

2. The rear tip extender of claim 1, wherein the trailing end (206, 436, 526, 626) of the first tooth bite edge (202, 432, 522, 622) and the trailing end (216, 446, 536, 636) of the second tooth bite edge (212, 442, 532, 632) are each an equal distance away from the distal end (190, 412) of the rear tip extender (104, 104', 400).

3. The rear tip extender of claim 1, wherein the leading end (204, 434, 524, 624) of the first tooth bite edge (202, 432, 522, 622) is closer to the distal end (190, 412) of the rear tip extender (104, 104', 400) than is the trailing end (206, 436, 526, 626) of the first tooth bite edge (202, 432, 522, 622).

4. The rear tip extender of claim 1, wherein the first tooth (180) comprises a first tooth base (203) secured to the cavity sidewall (196) and the second tooth (182) comprises a second tooth base (213) secured to the cavity sidewall, and the first tooth base and the second tooth base are each an equal distance (X) away from the distal end of the rear tip extender.

5. The rear tip extender of claim 1, wherein the plurality of teeth further comprises a third tooth (230, 540, 640) spaced a distance apart from the second tooth (182, 530, 630), where the third tooth comprises a third tooth bite edge (232, 542, 632) offset radially inward from the cavity sidewall, and the leading end (234, 544, 634) of the third tooth bite edge and the leading end (214, 534, 634) of the second tooth bite edge (212, 532, 632) are each an equal distance away (X) from the distal end of the rear tip extender.

6. The rear tip extender of claim 5, wherein the second tooth (182) comprises a second tooth base (213) secured to the cavity sidewall (196) and the third tooth (230) comprises a third tooth base (233) secured to the cavity sidewall, and the second tooth base and the third tooth base are each an equal distance away (X) from the distal end of the rear tip extender.

7. The rear tip extender of claim 1, wherein an exterior surface of the rear tip extender (104, 104', 400) extends from the distal end (190, 412) to a proximal end (192, 414), and the exterior surface comprises a tapered section (416) that converges from a diameter measured at the distal end to a narrower diameter measured at the proximal end.

8. The rear tip extender of claim 7, wherein the proximal end (192, 414) of the rear tip extender is a hemi-spherical proximal end.

9. The rear tip extender of claim 1, further comprising:
an annular lock ring (406, 506, 606) integrated in the cavity sidewall (422), and the plurality of teeth (430, 440, 520, 530, 540, 620, 630, 640) extend radially inward from the annular lock ring and are positioned along the annular lock ring to orient the plurality of teeth to extend radially inward from the cavity sidewall.

10. The rear tip extender of claim 9, wherein the annular lock ring has an axial length measured between a distal end (452, 552, 652) and a proximal end (454, 554, 654) of the annular lock ring, and the trailing end (436, 526, 626) of the first tooth bite edge (432, 522, 622) and the trailing end (446, 536, 636) of the second tooth bite edge (442, 532, 632) are each located at the proximal end of the annular lock ring.

11. The rear tip extender of claim 10, wherein the plurality of teeth further comprises a third tooth (540, 640) spaced a distance apart from the second tooth (530, 630), where the third tooth comprises a third tooth bite edge (542, 642) offset radially inward from the annular lock ring (506, 606), and the leading end (544, 644) of the third tooth bite edge and the leading end (534, 634) of the second tooth bite edge (532, 632) are each an equal distance away from the distal end (552, 652) of the annular lock ring.

12. The rear tip extender of claim 10, wherein the annular lock ring further comprises a first slot (460, 560, 660) formed in a wall (450, 550, 650) of the annular lock ring (406, 506, 606), with the first slot located between the first tooth (430, 520, 620) and the second tooth (440, 530, 630) and extending through the proximal end (454, 554, 654) of the annular lock ring a partial distance toward the distal end (452, 552, 652) of the annular lock ring.

13. The rear tip extender of claim 11, wherein the annular lock ring further comprises:
a first slot (570, 670) formed entirely through a wall (550, 650) of the annular lock ring (506, 606) between the first tooth (520, 620) and the second tooth (530, 630);
a second slot (580, 680) formed entirely through the wall of the annular lock ring between the second tooth (530, 630) and the third tooth (540, 640); and
a third slot (560, 660) formed entirely through the wall of the annular lock ring between the third tooth (540, 640) and the first tooth (520, 620);
wherein the first slot, the second slot, and the third slot allow each of the first tooth, the second tooth, and the third tooth to move independently one from another.

14. The rear tip extender of claim 11, wherein the third tooth bite edge (642) and the second tooth bite edge (632) are each angled away from the distal end (652) and toward the proximal end (654) of the annular lock ring (606) at an angle of about 10 degrees.

15. The rear tip extender of claim 1, further comprising:
a snap ring (210, 420) projecting radially inward from the cavity sidewall (196, 422) and located between the plurality of teeth (180, 182, 220, 230, 430, 440, 520, 530, 540, 620, 630, 640) and the distal end (190, 412) of the rear tip extender (104', 400);
wherein the snap ring is disposed around an entire circumference of the cavity sidewall.

16. The rear tip extender of claim 15, wherein the snap ring is rectangular in cross-sectional profile and projects at a right angle a radial height inward from the cavity sidewall (196), and the snap ring radial height away from the cavity sidewall is shorter than an axial length (L) of the snap ring longitudinally along the cavity sidewall.

17. The rear tip extender of claim 1, wherein the first tooth bite edge (202, 432, 522, 622) and the second tooth bite edge (212, 442, 532, 632) are each oriented to have an identical pitch.

18. The rear tip extender of claim 11, wherein the first tooth bite edge (522, 622), the second tooth bite edge (532, 632), and the third tooth bite edge (542, 642) are each oriented to have an identical pitch.

19. The rear tip extender of claim 11, wherein each of the teeth (620, 630, 640) comprises a stand off (655) that defines a proximal end (654) of the annular lock ring (606).
